# EUROPEAN PATENT APPLICATION

(11) **EP 2 612 611 A1**
(43) Date of publication of application: **10.07.2013**
(21) Application number: 13162625.1
(22) Date of filing: 03.12.2009
(51) Int. Cl.: A61B 17/86

(54) **Bone screw**

(62) Divisional of application: 09177910.8
(71) Applicant: Biedermann Technologies GmbH & Co. KG, 78166 Donaueschingen (DE)
(72) Inventor: Biedermann, Lutz, 78048 VS-Villingen (DE); Matthis, Wilfried, 79367 Weisweil (DE); Harms, Jürgen, 76227 Karlsruhe (DE)
(74) Representative: Prüfer & Partner GbR European Patent Attorneys

(57) **Abstract**

A bone screw including a tubular body (1) with a bone thread (5) in at least a portion of its outer wall, the tubular body having an open first end (2) and an open second end (3); wherein at the open first end (2) a plurality of cutting teeth (4) is provided and wherein at the second end (3) a structure (31, 310) for engagement with a screwing-in tool (100) is provided.

## Description

The invention relates to a bone screw. In particular, the invention relates to a bone screw which can be used as a fusion screw. This screw promotes fusion in the surrounding bone.

A bone screw of this kind is known from US 2004/0015172 A1. This bone screw has a tubular thread section with a bone thread and with a plurality of recesses in its wall. A head and a tip can be connected to the tubular thread section. In use, the tubular portion can be filled with bone material or other growth-promoting material and then the tip and/or the head are connected to the tubular portion. First, a core hole is prepared, then the screw is inserted into the core hole and screwed into the bone. After a certain period, fusion between the screw and the bone takes place. The screw can act as a traction element to connect shattered or split off parts of bones together by means of the screw. To avoid the preparation of a core hole, the tip can be self-cutting.

US 2004/0122431 describes a bone screw of the kind mentioned above, however without a head. Such a bone screw can be used for example together with a marrow nail. US 2005/0055026 A1 describes a bone screw of the type mentioned above wherein a head which is a separate part can be connected to a tubular threaded shank. The head is configured to be connected to a stabilization rod. In one embodiment, the tubular threaded shank of the screw has at its end which is to be connected to the head slits for engagement with a screw driver.

Further, so-called injection screws, for example such as described in WO 01/26568 A1 are known which are used to inject bone cement or other liquid or pasty material into the bone. This kind of screw has a relatively thin cannula entending within a bone screw. The cannula is not suitable for being filled with bone chips or bone graft.

It is the object of the invention to provide a bone screw which can be quickly screwed into the bone without using a prepared core hole.

The object is solved by a bone screw according to claim 1. Further developments are given in the dependent claims.

The bone screw according to the invention is self-filling. When it is screwed into the bone the cutting teeth engage the bone and create a hole. The bone material from the location at which the screw is inserted fills the cavity of the tubular body. Hence, the preparation of a core hole is not necessary. It is not necessary to pre-fill the tubular body with bone material. Therefore, the procedure of implanting the bone screw is less time consuming than in a case where the bone screw has to be pre-filled and inserted into a core hole prepared in advance. Furthermore, the bone material has a more intact structure compared to pre-filled bone material which could enhance the healing process.

The bone screw is particularly suitable for strong healthy bones and for weak osteoporotic bones, since the structural damage which occurs when screwing-in the bone screw can be kept to a minimum.

By using a guide wire procedure for insertion the bone screw can be used in minimally invasive surgery.

Further features and advantages of the invention will become apparent from the description of various embodiments of the invention by means of the accompanying drawings.

In the drawings:
- Fig. 1: shows a perspective view of a bone screw according to a first embodiment.
- Fig. 2: shows a top view of the bone screw according to Fig. 1.
- Fig. 3: shows a sectional view of a bone screw according to Fig. 1, the section being taken in a plane containing the longitudinal axis L of the bone screw.
- Fig. 4: shows a side view of the bone screw of Fig. 1.
- Fig. 5: shows a perspective view of a bone screw according to a second embodiment.
- Fig. 6: shows a top view of the bone screw of Fig. 5.
- Fig. 7: shows a sectional view of the bone screw of Fig. 5, the section being taken in a plane containing the longitudinal axis L of the bone screw.
- Fig. 8: shows a side view of the bone screw of Fig. 5.
- Fig. 9: shows a perspective view of the bone screw of Figs. 5 to 8 together with a guide wire and a tool for insertion.
- Fig. 10: shows a sectional view of the tool which comprises an engagement structure.

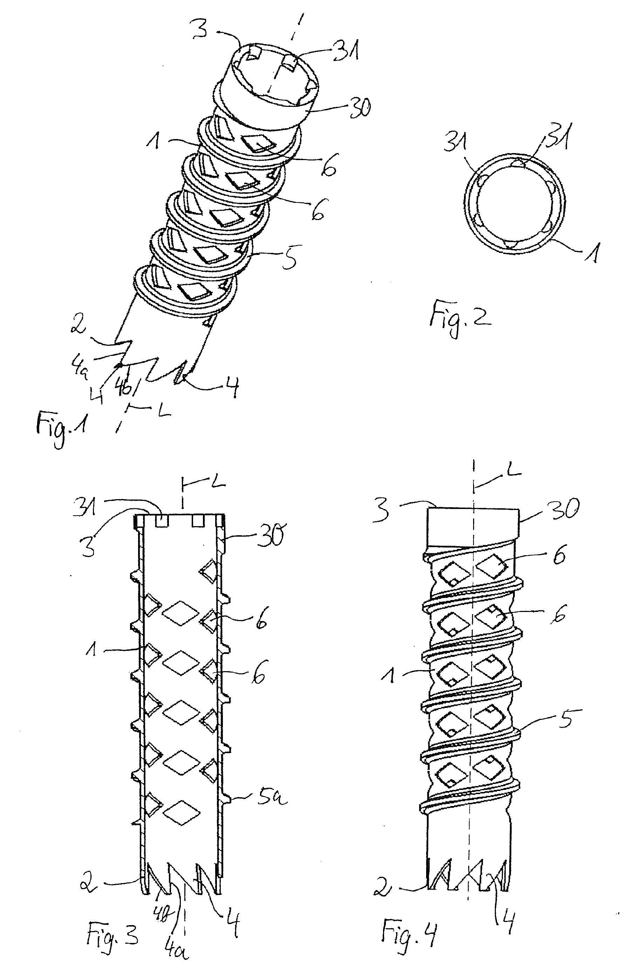

A bone screw according to a first embodiment as shown in Figs. 1 to 4 comprises a tubular body 1 with an open first end 2 and an open second end 3 and a central longitudinal axis L. At the first end 2 the free edge of the tubular body comprises a plurality of cutting teeth 4 which are configured to cut a hole into the bone. In a portion of its outer wall, as shown in the embodiment this portion is close to the first end 2, the tubular body comprises a so-called bone thread 5. The bone thread 5 is configured to cut into the bone when the bone screw is screwed-in into the bone. Although, the Figures show a bone thread with a substantially saw-tooth shape, all kind of known bone threads can be used. The cutting teeth 4 extend from the edge of first end 2 coaxially to the longitudinal axis L. In the embodiment shown, they are substantially saw-tooth shaped. This shape includes a steep flank 4a which extends under an angle of larger than 45° with respect to a circumferential line along the free edge and a more shallow flank 4b which extends under an angle of greater than 0° up to 45° with respect to the free edge. In order ease the insertion of the screw, the steep flanks are oriented all in the same circumferential direction. The cutting teeth are configured to cut a ring-shaped hole into the bone. Within the hole, bone material remains. The bone material which remains in-between the cutting teeth is accommodated in the tubular body. It can consist either of chips or it can be a plug-like coherent mass or both.

The thickness of the cutting teeth in a radial direction can be larger than a thickness of the wall of the tubular body (not shown). This results in providing an enlarged space within the tubular body to accommodate the bone material.

The height, the shape and the number of the cutting teeth can vary. All kinds of cutting teeth are suitable which are configured to cut the bone so that bone material remains inside the tubular body.

In a wall of the tubular body, a plurality of openings 6 are provided to allow in-growth of bone material and vessels from the surrounding of the bone screw. The opening are shown as diamond-shaped and extend completely through the wall of the tubular body 1. They are located between the crests 5a of the bone thread 5. Any other variations of the shapes and locations of the openings 6 are conceivable.

Adjacent the second open end 3 a section 30 of the tubular body 1 is formed which is non-threaded. The section 30 comprises at its inner wall a plurality of recesses 31 equally spaced in a circumferential direction which form an engagement structure for a screw driver or another tool. The recesses 31 extend from the edge of the open end 3 to a certain distance therefrom. The wall thickness of the tubular body 1 in the section 30 having the engagement structure may be enhanced compared to the wall thickness of the remaining tubular body 1. The recesses shown in the Figures form a star-like engagement structure for a star screw driver. However, any other shape of the engagement structure such as a hexagon socket or square socket or any other polygon socket is conceivable.

Although the tubular body is shown to be cylindrically-shaped, other shapes are conceivable. For example, the tubular body 1 may have close to the first end 2 a reversed tapered section tapering away from the first end 2. The cavity provided by the tubular body has a volume which is suitable for accommodating bone material. The wall thickness of the tubular body is preferably smaller than about 15% of the screw core diameter.

All parts of the bone screw are made of a body compatible material such as a body compatible metal, for example stainless steel or titanium, a body compatible metal alloy, for example Nitinol or a body compatible plastic material, for example PEEK.

In addition, the tubular body or the other parts of the bone screw can be coated with an in-growth promoting material or can be roughened to enhance in-growth of bone or vessels.

In use, the bone screw according to the first embodiment is inserted into the bone by engaging the engagement structure 31 with a screw driver. The cutting teeth 4 generate the hole for the screw and the bone thread 5 facilitates further advancement of the bone screw into the hole. Bone material which is scraped-off by the cutting teeth 4 fills the interior of the tubular body 1. After a certain time period, the bone material in the tubular body fuses with bone material surrounding the bone screw so that the bone screw is rigidly connected to the bone.

Since the bone screw does not have a head it is not used as a tension screw which is fused for reposition of bone fragments. The bone screw according to this embodiment is used for the immobilization of joints, for example for the immobilization of facet joints.

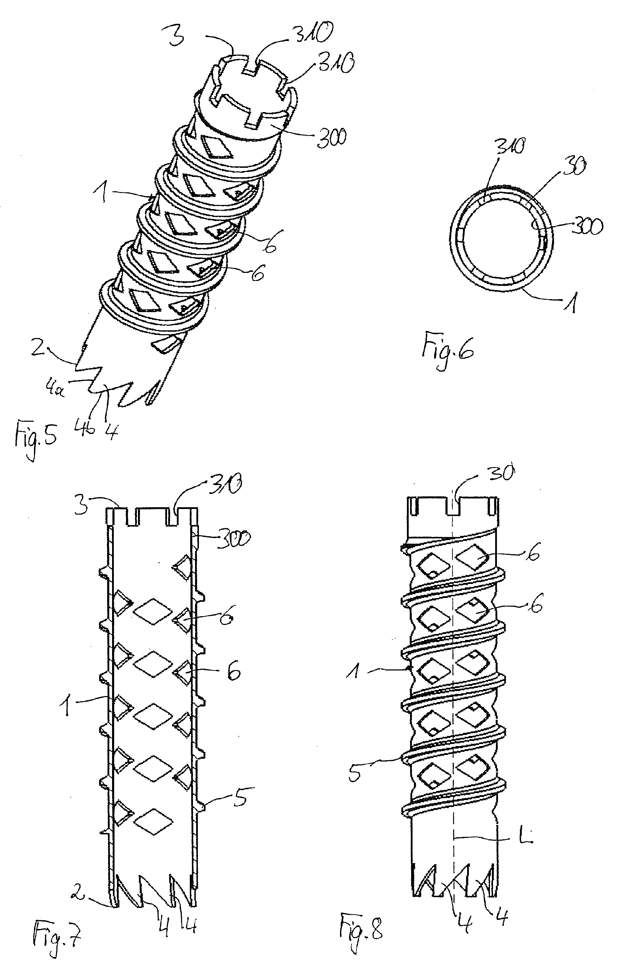

In Figs. 5 to 8 a second embodiment of the bone screw is shown. The second embodiment differs from the first embodiment in the design of the engagement structure for the tool. All other portions are similar to the first embodiment and are designated with the same reference numerals. The description thereof is not repeated.

The bone screw according to the second embodiment has adjacent its open second end 3 a non-threaded portion 300 which comprises a plurality of slits 310 extending from the edge of the open end to a certain distance in longitudinal direction of the tubular body 1. The slits 310 extend completely through the wall so that they form an engagement structure which can be engaged with a corresponding engagement structure of a tool, for example with pins of a tool. The shape of the slits 310 is shown as rectangular. However, any other shape is conceivable which allows to transmit torque when the engagement structure of the tool engages the slits. The number and dimension of the slits can vary.

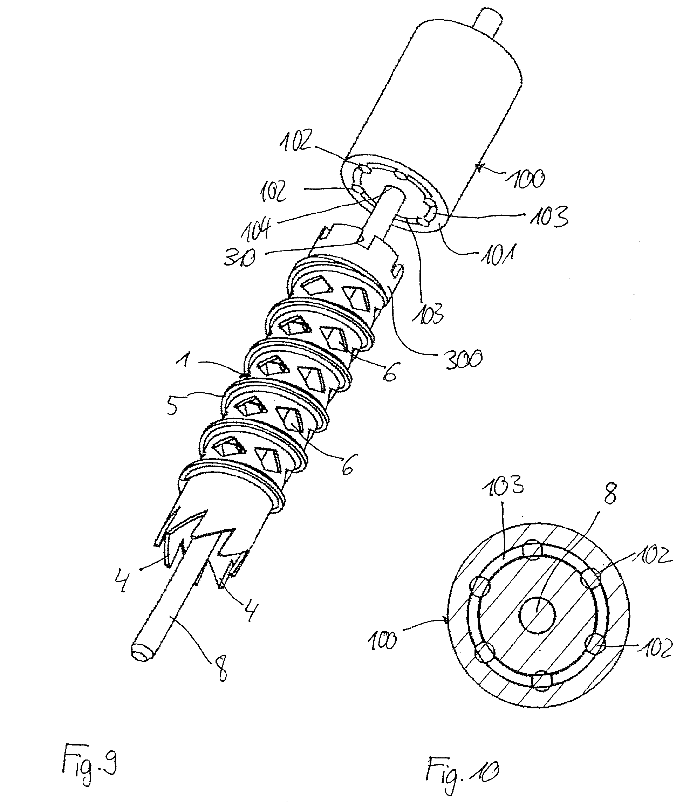

Figs. 9 and 10 show the bone screw according to the second embodiment together with a tool 100 and a guide wire 8. The tool 100 is for example a cylinder which is adapted to cooperate with the engagement structure of the bone screw. At the front face 101 a plurality of pins 102 are provided which are configured so as to engage the recesses 310 of the engagement structure of the bone screw. Further, a groove 103 is provided in which the free edge of the open end 3 of the tubular body of the bone screw can be inserted to facilitate the appliance of the tool 100. The pins 102 are located at equal distances in the groove and do not or do not much project out of the front face 101. The tool 100 further comprises a coaxial guide hole 104 for guiding the guide wire 8 therethrough. The length of the tool 100 is such that the guide wire 8 is guided by the tool 100 in a stabile manner.

Although the tool is shown to be cylindrical, it can have any other shape and any other engagement structure which cooperates with the engagement structure of the bone screw. For example, the engagement structure can be designed so as to engage the engagement structure of the bone screw according to the first embodiment.

The bone screw with the tool and the guide wire as shown in Figs. 9 and 10 can be used in minimally invasive surgery (MIS). First, the guide wire which is guided by the tool 100 is inserted into the tubular body 1 as shown in Fig. 9. Then, the guide wire 8 is introduced through the skin of the patient and advanced through the tissue until it reaches the position where the bone screw has to be placed. Then, the guide wire is inserted into the bone to the appropriate direction and depth. Then, the bone screw is guided along the guide wire 8 extending therethrough until it reaches the surface of the bone. Next, the bone screw is screwed into the bone guided by the guide wire by rotating the tool 100. The cutting teeth 4 generate the hole for the screw and the bone thread 5 facilitates further advancement of the bone screw into the hole.

Bone material which is scraped-off by the cutting teeth 4 fills the interior of the tubular body 1. When the bone screw is finally implanted, the guide wire is retracted. After a certain time period, the bone material in the tubular body fuses with bone material surrounding the bone screw so that the bone screw is rigidly connected to the bone. The bone screw can be used as the bone screw according to the first embodiment particularly for stabilizing joints.

## Claims

1. Bone screw including
a tubular body (1) with a bone thread (5) in at least a portion of its outer wall, the tubular body having an open first end (2) and an open second end (3);
wherein at the open first end (2) a plurality of cutting teeth (4) is provided and
wherein at the second end (3) a structure (31, 310) for engagement with a screwing-in tool (100) is provided.

2. Bone screw according to claim 1, wherein the cutting teeth (4) extend coaxially to the central axis (L) of the tubular body.

3. Bone screw according to claim 1 or 2, wherein the cutting teeth (4) are configured to cut a hole when the tubular body (1) is screwed into the bone.

4. Bone screw according to one of claims 1 to 3, wherein the cutting teeth (4) have a substantially saw tooth shape.

5. Bone screw according to one of claims 1 to 4, wherein the cutting teeth (4) are thicker in a radial direction compared to the wall of the tubular body.

6. Bone screw according to one of claims 1 to 5, wherein the cutting teeth (4) form an edge of the open first end (2).

7. Bone screw according to one of claims 1 to 6, wherein the wall thickness of the tubular body is smaller than about 15% of the outer diameter of the tubular body.

8. Bone screw according to one of claims 1 to 7, wherein the engagement structure comprises a plurality of recesses (31) extending from the open second end (3) through the wall of the tubular body substantially coaxially to a central longitudinal axis (L) of the tubular body.

9. Bone screw according to one of claims 1 to 8, wherein the engagement structure comrises a polygon recess or is a star-like recess (31) extending from the open second end (3) into the inner wall of the tubular body.

10. Bone screw according to one of claims 1 to 9, wherein the wall of the tubular body comprises a plurality of openings (6).

11. Bone screw according to one of claims 1 to 10, wherein the tubular body comprises a non threaded section.

12. Bone screw according to one of claims 1 to 11, wherein a guide wire (8) is provided which is insertable into the tubular body for guiding the insertion of the bone screw.

13. Bone screw according to one of claims 1 to 12, wherein the tubular body (1) is cylindrical.

14. Tool for inserting the bone screw according to one of claims 1 to 13, wherein the tool comprises an engagement structure (102, 103) which engages the engagement structure (31, 310) of the bone screw and which is configured to guide a guide wire.

15. Tool according to claim 14, wherein the tool comprises a guide hole (104) for guiding the guide wire therethrough.
